(19) Europäisches Patentamt
European Patent Office
Office européen des brevets

(11) Numéro de publication: **0 252 829 A1**

# (12) DEMANDE DE BREVET EUROPEEN

(21) Numéro de dépôt: **87401574.6**

(22) Date de dépôt: **06.07.87**

(51) Int. Cl.[4]: **A 61 K 39/00**
G 01 N 33/53

(30) Priorité: **07.07.86 FR 8609842**

(43) Date de publication de la demande:
**13.01.88 Bulletin 88/02**

(84) Etats contractants désignés:
**AT BE CH DE ES FR GB GR IT LI LU NL SE**

(71) Demandeur: **INSTITUT PASTEUR**
**28, rue du Docteur Roux**
**F-75724 Paris Cédex 15 (FR)**

**INSTITUT PASTEUR DE LILLE**
**Rue du Professeur A. Calmette BP 245**
**F-59019 Lille Cédex (FR)**

**INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur: **Montreuil, Jean**
**145, rue Jules Boucly**
**F-59650 Villeneuve D'Asq (FR)**

**Spik, Geneviève**
**résidence du Moulin 39 rue de la Pilaterie**
**F-59700 Marçq en Baroeul (FR)**

**Capron, André**
**58 rue du Capitaine Jasmin**
**F-59133 Phalempin (FR)**

**Dissous, Colette**
**7 Allée des Symphorines**
**F-59262 Sainghin en Melantois (FR)**

**Grzych, Jean-Marie**
**1 rue Meunier Résidence des Tilleuls&Apt. 202**
**F-59700 Marçq en Baroeul (FR)**

(74) Mandataire: **Ores, Irène et al**
**CABINET ORES 6, Avenue de Messine**
**F-75008 Paris (FR)**

(54) **Fraction immunogène active à l'égard des bilharzioses, sa préparation, compositions immunisantes la contenant.**

(57) La présente invention est relative à une fraction immunogène active à l'égard des bilharzioses.

Cette glycoprotéine extraite de l'hémocyanine d'un mollusque, se caractérise en ce que sa fraction glucidique représente 5 à 25 % de la molécule de glycoprotéine et en ce que la composition molaire de ladite fraction glucidique (calculée sur la base de 3 résidus de mannose) est sensiblement la suivante :

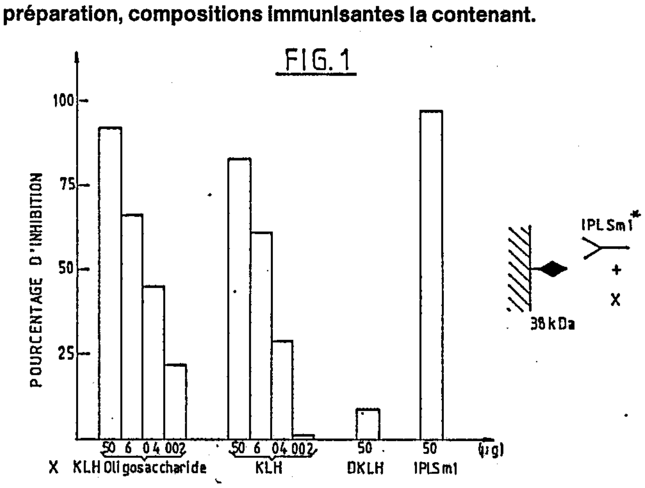


Application : compositions immunisantes à l'égard des bilharzioses.

EP 0 252 829 A1

**Description**

FRACTION IMMUNOGENE ACTIVE A L'EGARD DES BILHARZIOSES, SA PREPARATION, COMPOSITIONS IMMUNISANTES LA CONTENANT.

Le Brevet français N° 8606281 du 30 Avril 1986 décrit une glycoprotéine extraite d'hémocyanine de Megathura crenulata, désignée en abrégé "KLH" et son épitope oligosacharidique. Il est montré dans ce Brevet que ladite glycoprotéine présente les mêmes propriétés antigéniques que l'antigène 38 KD isolé de la surface des schistosomules de Schistosoma mansoni et que l'antigène 38 KD du schistosomule et l'hémocyanine de Megathura crenulata contiennent tous deux le même oligosaccharide qui est l'épitope oligosaccharidique précité, lequel est reconnu par un anticorps monoclonal protecteur comme étant l'épitope responsable du pouvoir anti-génique de l'antigène 38 KD de schistosomule et de la KLH, l'anticorps monoclonal protecteur étant obtenu à partir de l'hybridome IPL Sm1 identifié dans la Demande de Brevet précitée.

La présente invention a pour but de déterminer la structure chimique de la glycoprotéine et de son épitope oligosaccharidique, qui font l'objet du Brevet français précité et de permettre d'en réaliser la synthèse.

La présente invention a pour objet une glycoprotéine extraite de KLH (hémocyanine de Megathura crenulata) qui est caractérisée en ce que sa fraction glucidique représente 5 à 5,25% de la molécule de glycoprotéine et en ce que la composition molaire de ladite fraction glucidique (calculée sur la base de 3 résidus de mannose) est sensiblement la suivante :

| | Résidus monosaccharidiques |
|---|---|
| Mannose | 3 |
| Galactose | 3 à 4 |
| Fucose | 2 à 3 |
| Glucose | 2 ± 0,2 |
| Xylose | 0,5 - 1 |
| N-acétylglucosamine | 3 - 4 |
| N-acétylgalactosamine | 2 ± 0,3 |

Les monosaccharides qui constituent la fraction glucidique de la KLH ont été identifiés et dosés par chromatographie en phase gazeuse associée à la spectrométrie de masse, ce qui a permis de constater l'absence de monosaccharides O-méthylés dans ladite fraction glucidique.

La présente invention a également pour objet la fraction glycannique isolée de la glycoprotéine précitée, extraite de KLH par protéolyse par la pronase et purification du produit d'hydrolyse par chromatographie de gel-filtration pour recueillir le produit du pic majeur obtenu, dont la composition en glucides (calculée sur la base de 3 résidus de mannose) est sensiblement la suivante :

| | Nbre/résidus monosaccharidiques |
|---|---|
| Mannose | 3 |
| Galactose | 4 |
| Fucose | 1,6 |
| Glucose | 4 |
| Xylose | 0,23 |
| N-acétylglucosamine | 3,3 |
| N-acétylgalactosamine | 2 |

La présente invention a en outre pour objet un glycanne de synthèse sensiblement identique au glycanne extrait de la KLH et au glycanne de l'antigène 38 KD de S. mansoni, qui est caractérisé en ce qu'il présente au moins l'une des structures primaires suivantes :

Glycannes de N-glycosylprotéines

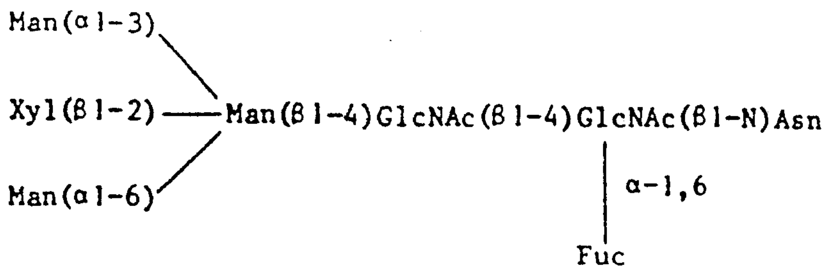

(I)

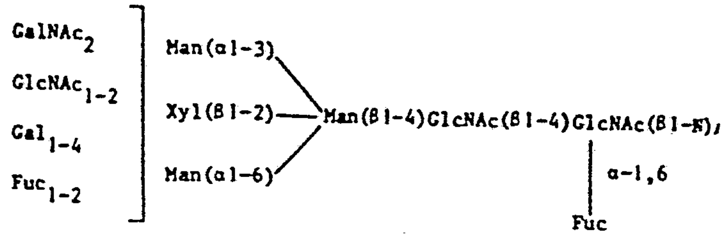

(II)

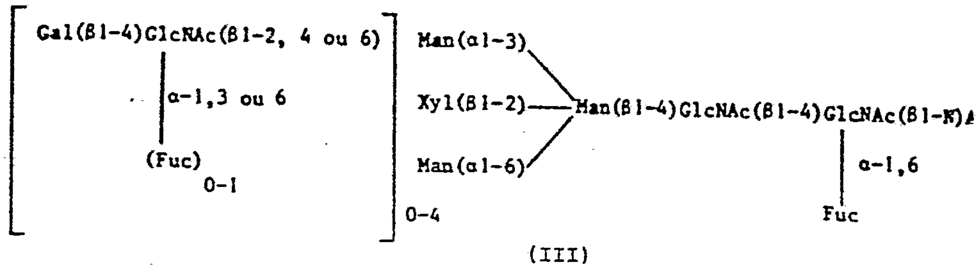

(III)

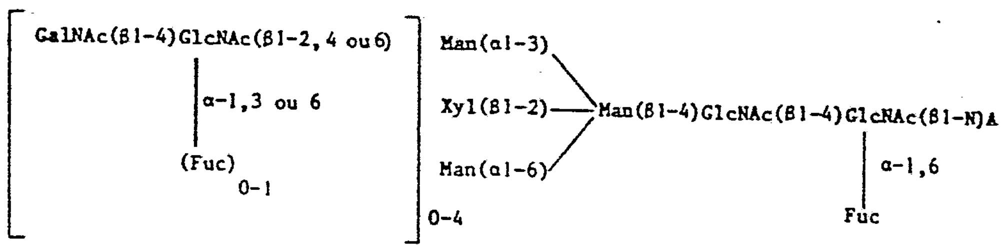

(IV)

Glycannes de O-glycosylprotéines.

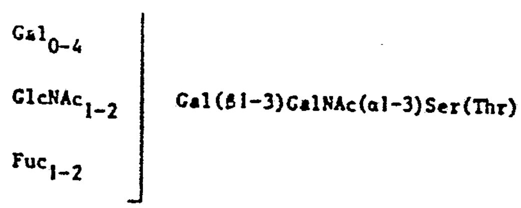

(V)

L'activité antigénique des glycannes isolés de KLH ou obtenus par synthèse a été évaluée à l'aide de la sonde monoclonale spécifique de la molécule 38 KD, à savoir les anticorps IPL SmI utilisés dans le Brevet français mentionné plus haut par la technique d'inhibition de la fixation de l'anticorps IPL Sm1 marqué à l'iode 125, respectivement sur la glycoprotéine extraite de KLH et sur le glycanne de synthèse conforme à la présente invention, par les sérums d'animaux immunisés par la KLH. Les résultats obtenus sont présentés à la Figure 1 annexée et font apparaître que les anticorps produits au cours d'une telle immunisation induisent des taux d'inhibition identiques à ceux observés respectivement pour les sérums d'infection, pour l'anticorps IPL Sm1 "froid", pour la KLH purifiée ou pour l'antigène cible 38 KD.

Cette démonstration de l'activité inhibitrice de la fraction glycannique identifiée conformément à la présente invention, vis-à-vis de l'anticorps monoclonal dirigé contre l'antigène 38 KD de Schistosoma mansoni et contre la glycoprotéine extraite de l'hémocyanine de Megathura crenulata, vient confirmer de façon définitive les travaux précédents qui concluaient au fait que l'épitope de l'antigène de surface 38 KD devait être au moins en partie glycannique [FEBS LETTERS, DISSOUS et CAPRON, 162 (1983) p. 355-359 et MOLECULAR AND BIOCHEMICAL PARASITOLOGY, DISSOUS et Al., 16 (1985), p. 277-288].

La présente invention a, en conséquence, également pour objet des sérums protecteurs à l'égard des

infections par Schistosoma mansoni, des compositions immunogènes, des vaccins, des agents immunisants et des réactifs de diagnostic qui sont caractérisés en ce que leur constituant actif est la glycoprotéine et/ou le glycanne identifié(s) dans ce qui précède.

La présente invention a en outre pour objet un procédé de préparation de la glycoprotéine identifiée plus haut, qui consiste à soumettre de l'hémocyanine de Megathura crenu lata à au moins un traitement de protéolyse par la pronase et à isoler le produit de la protéolyse par précipitation par un agent précipitant approprié, tel que l'éthanol notamment.

Selon un mode de mise en oeuvre préféré du procédé conforme à la présente invention, le produit de la protéolyse isolé par précipitation, est soumis à un traitement de purification approprié, de préférence à un traitement de purification par chromatographie de gel-filtration, après quoi la fraction éluée présentant le pic majeur est isolée par précipitation, pour fournir une fraction glycopeptidique purifiée.

Comme indiqué plus haut, la composition en glucides des fractions ainsi isolées a été déterminée qualitativement et quantitativement par chromatographie en phase gazeuse couplée à la spectrométrie de masse.

Conformément à l'invention, les glycannes liés O-glycosidiquement sont libérés par la méthode de $\beta$-élimination sous forme d'oligosaccharides-alditols, la liaison N-glycosidique n'est pas touchée.

La réaction est effectuée sur 93mg de glycopeptides pronasiques.

La solution finale résultant de la $\beta$-élimination est fractionnée sur une colonne de Biogel P2 équilibrée dans l'eau. Les glycopeptides alcali-stables dépassant 1800 D, masse moléculaire d'exclusion du Biogel P2, sont élués au volume mort de la colonne, tandis que les O-glycannes sont retardés.

Le repérage des fractions éluées est fait par chromatographie en couche mince. On isole ainsi 4 fractions dont la fraction A représente la majeure partie (1/3) et correspond aux glycopeptides alcali-stables. Cette fraction A est séparée par passage sur colonne de Con A Sépharose 4B en 3 sous-fractions $A_1$ , $A_2$ , $A_3$ dont seule la fraction $A_1$ conserve une activité biologique comparable à celle de la fraction A. Cette fraction $A_1$ isolée de l'hémocyanine de Megathura crenulata est caractérisée par la formule suivante :

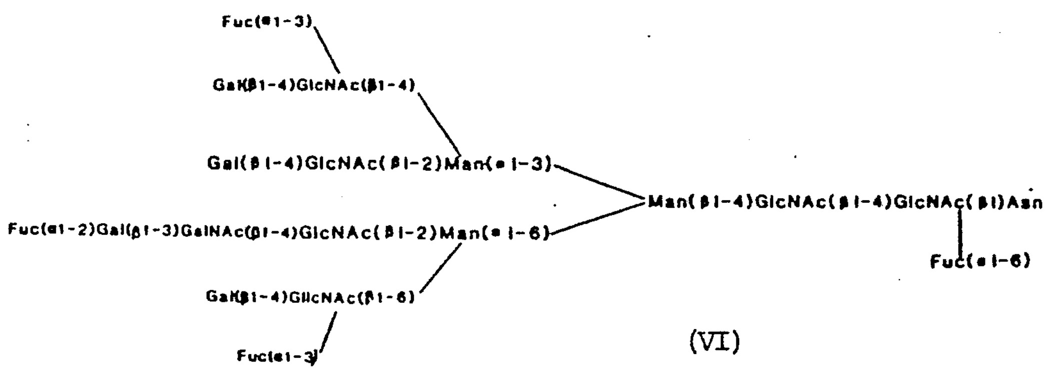

(VI)

L'activité antigénique des fractions glycopeptidiques extraites de l'hémocyanine de Megathura crenulata, identifiées plus haut, et celle des glycannes de synthèse qui répondent à l'une des structures primaires des formules I à IV ont été évaluées, entre autres, à l'aide du test d'inhibition évoqué plus haut.

Outre les application énoncées plus haut, les glycopeptides isolés de protéolysats de KLH ou les glycannes libérés par hydrolyse chimique ou enzymatique de KLH ou encore préparés par synthèse organique, conformément à la présente invention, peuvent être mis en oeuvre pour la préparation de néoglycoprotéines, par condensation desdits glycopeptides ou glycannes avec des peptides naturels ou de synthèse.

Selon un mode de mise en oeuvre avantageux de ce procédé, la condensation desdits glycopeptides ou glycannes avec des peptides appropriés est réalisée par couplage d'oligosaccharides et de protéines.

Selon un autre mode de mise oeuvre avantageux de ce procédé, la préparation des néoglycoprotéines est réalisée par couplage de glycopeptides et de protéines.

Comme exemples de peptides pouvant être condensés avec les glycannes conformes à la présente invention, on peut citer l'anatoxine tétanique et/ou des peptides de synthèse dérivés de la protéine 28kDa.

Les néoglycoprotéines ainsi obtenues présentent l'intérêt de grouper dans une structure chimique unique, deux activités antigéniques complémentaires, à savoir l'activité antigénique du glycanne conforme à la présente invention et l'activité antigénique de la fraction peptidique associée audit glycanne pour former la néoglycoprotéine désirée.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de préparation des glycannes conformes à l'invention par extraction à partir de KLH, de mise en évidence de l'activité antigénique desdits glycannes et de préparation de néoglycoprotéines également conforme à la présente invention.

Il doit être bien entendu, toutefois, que ces exemples sont donnés uniquement à titre d'illustration de l'objet

de l'invention, dont ils ne constituent en aucune manière une limitation.

EXEMPLES

EXEMPLE 1 - Préparation de glycopeptides conformes à l'invention par extraction à partir de KLH.

1. De l'hémocyanine de Megathura crenulata (KLH) est mise en contact avec de la pronase dans un rapport enzyme/substrat de l'ordre de 1/50 pour réaliser la pro téolyse de la KLH ; la réaction est réalisée à une température de 40°C, à pH 8,2, pendant une durée de 48 heures. Le produit de la protéolyse est isolé par précipitation par de l'éthanol.

La réaction de protéolyse suivie de précipitation, est répétée deux fois encore.

2. Le produit final obtenu à l'issue du 3ème processus de protéolyse est purifié par chromatographie de gelfiltration sur "Biogel" P-2.

3. Le produit élué dans le pic majeur contient le glycanne actif encore lié à une courte chaîne peptidique.

Sa composition en glucides est vérifiée par chromatographie en phase gazeuse couplée à la spectrométrie de masse.

Son activité antigénique est vérifiée par la technique radioimmunologique d'inhibition de la fixation de l'anticorps monoclonal IPL Sm1 sur le glycanne, comme décrit pour la KLH et l'antigène 38 KD dans le Brevet français No 86 06281 déjà cité.

EXEMPLE 2 - Préparation des glycannes conformes à l'invention par extraction à partir de KLH ou de glycopeptides de KLH>

Les glycannes liés N-glycosidiquement sont libérés par le procédé de Lee et Scocca (J. Biol. Chem., 247 (1972) 5753-5758) : une solution de KLH ou de ses glycopeptides dans NaOH M - $KBH_4$ M est maintenue à 100°C pendant 6 h. La réaction est arrêtée par l'addition, à 0°C, d'acide formique jusqu'à obtenir un pH de 4. La fraction glucidique est purifiée par gel-filtration sur Biogel P-2, l'élution étant réalisée par l'eau. La N-réacétylation des oligosaccharides en solution saturée de bicarbonate de sodium est effectuée par l'anhydride acétique dont l'action est poursuivie pendant 2 h. La fraction glucidique est ensuite purifiée par chromatographie sur Biogel P-2.

Les glycannes liés O-glycosidiquement sont spécifiquement libérés par $\beta$-élimination en traitant la KLH ou les glycopeptides en dérivant selon le protocole précédent à l'exception des conditions de l'attaque sodique qui se fait en présence de NaOH 0,1 M - $KBH_4$ 1M à 45°C pendant 24 h.

EXEMPLE 3 - Tests de caractérisation de l'activité antigénique des glycannes conformes à la présente invention.

1o : Technique d'inhibition de la fixation de l'anticorps IPLSml sur l'antigène 38 kDa du S. mansoni par les oligosaccharides extraits de KLH.

Cette technique radioimmunologique en phase solide est basée sur l'inhibition de la fixation de l'anticorps IPLSml marqué à l'iode 125 à son antigène cible par différents anticorps ou préparations antigéniques.

a) Préparation de la phase solide

Chaque alvéole de plaque de polyvinyle est traitée par 100 µl d'une solution à 10 µg/ml d'anticorps monoclonal $C_3$ -109 (anticorps anti-S. mansoni d'isotype IgM qui ne croise pas avec l'épitope de l'anticorps IPLSml). Cette méthodologie permet la fixation ultérieure de l'antigène 38 kD. Après 2 h de contact à 20°C, les plaques sont lavées 3 fois par 200 µl de Tampon phosphate 10 mM contenant 0,1 % de sérum albumine bovine (BSA), et saturées 30 minutes à 20°C par 200 µl d'une solution à 2 % de BSA en Tampon phosphate. Les plaques sont ensuite lavées 3 fois en tampon phosphate 10 mM 0,1 % BSA et traitées par 100 µl d'un extrait membranaire de schistosomule obtenu selon la technique décrite par DISSOUS et al. dans Mol. Biochem. Parasitol. (1981), 3, pages 215-225 (100 µl de solution antigénique à 100 µg/ml pour chaque alvéole). Après 2 h d'incubation à 37°C et 3 lavages par 200 µl de Tampon phosphate 10 mM 0,1 % BSA, les plaques sont prêtes pour la réaction d'inhibition.

b) Réaction d'inhibition

Cinquante microlitres d'anticorps IPLSml marqué à l'iode 125 (100.000 cpm dans 50 µl) et 50 µl des différentes fractions antigéniques (50 à 0,02 µg/alvéole) d'oligosaccharide de KLH, de KLH native ou 50 µg des témoins KLH déglycosylée (DKLH), anticorps IPLSml froid sont incubés 1 h à 37°C, puis 1 nuit à 4°C. Les plaques sont alors lavées 3 fois en Tampon phosphate 10 mM 0,1 % de BSA, et les alvéoles comptés séparément au compteur gamma.

c) Expression des résultats

Le pourcentage d'inhibition de fixation de l'anticorps IPLSml est évalué par rapport à un témoin négatif dans lequel les 50 µl d'IPLSml marqué sont incubés en présence de 50 µl de Tampon phosphate 10 mM 0,1 % de BSA, et est représenté à la Fig. 1 annexée.

2o : Technique d'inhibition de la fixation de l'anticorps IPLSml sur l'hémocyanine de Megathura crenulata par les oligosaccharides extraits de l'hémocyanine de Megathura crenulata.

Cette technique radioimmunologique en phase solide est directement inspirée de la méthode d'évaluation de l'inhibition de la fixation de l'anticorps IPLSml à son antigène cible 38 kD. Dans ce cas particulier, la phase solide est constituée par des plaques de polyvinyle préalablement traitées par de la KLH.

a) Phase solide

Chaque alvéole de plaque de polyvinyle est traitée par 100 µl d'une solution de KLH à 10 µg/ml en Tampon phosphate 10 mM à 0,1 % de BSA pendant 2 h à 20°C. Les plaques sont ensuite lavées 3 fois en Tampon phosphate 10 mM à 0,1 % de BSA et saturées par une solution à 2% de BSA dans le Tampon phosphate 10 mM, 30 minutes à 20°C (200 µl par alvéole). L'excès de BSA est éliminé grâce à 3 lavages (200 µl par alvéole) en Tampon phosphate 10 mM 0,1 % de BSA.

b) Réaction d'inhibition

Elle est réalisée dans les mêmes conditions que celles définies dans le cas du test d'inhibition de la fixation de l'anticorps IPLSml à son antigène 38 kD décrit au 1° du présent Exemple, et ses résultats sont représentés à la Fig. 2 annexée.

EXEMPLE 4 - Préparation de néoglycoprotéines conformes à la présente invention.

I - Par couplage d'oligosaccharides et de protéines.

Le couplage oligosaccharide-protéine peut être réalisé selon les protocoles expérimentaux suivants, la protéine utilisée étant soit l'anatoxine tétanique, soit des fragments peptidiques de synthèse de l'antigène 38 kD .

1o) Méthode de Gray (Gray G.R., Methods in Enzymology, 1979, Colowick and Kaplan ed., Acad. Press, p. 155-162).

Le principe de la méthode repose sur le fait que le cyanoborohydrure de sodium réduit aisément les bases de Schiff résultant de la condensation d'un oligosaccharide avec une amine primaire, tout en étant sans action sur les fonctions carbonyl.

$$R_1\text{-CHO} \rightleftarrows R\text{-CH} = \overset{+}{N}H\text{-}R_2 \xrightarrow{BH_3CN^-} R_1\text{-}CH_2\text{-}NH\text{-}R_2$$

Le protocole expérimental comprend les étapes suivantes : 0,5 µmol de protide, 150 µmol d'oligosaccharide et 0,8 mmol de cyanoborohydrure de sodium sont dissous dans 2,5 ml de tampon phosphate de potassium 0,2 M (pH 8) et la solution est maintenue à 37°C pendant 10 jours.

La solution est ensuite dialysée contre de l'eau distillée, puis soumise à une chromatographie sur Bio-gel P-6.

Le nombre de résidus oligosaccharidiques couplés sur la protéine est ensuite déterminé par un dosage colorimétrique. Il est généralement de l'ordre de 15 à 20 glycannes par molécule de protéine.

2o) Méthode de Zopf et al. (Zopf, D.A., Tsai C-M et Ginsburg V., Methods in Enzymology, 1978, Colowick and Kaplan ed. Acad. Press, p. 163-169).

Cette méthode a le mérite d'être applicable à de faibles quantités de substrats. L'oligosaccharide est tout d'abord couplé à la β-(p-aminophényl) éthylamine, en présence de borohydrure de sodium. Le dérivé oligosaccharidephénéthylamine est ensuite couplé au protide par l'intermédiaire d'un diazoïque.

a) Préparation de l'oligosaccharide-phénéthylamine

10 mg d'oligosaccharide sont ajoutés à 0,1 ml de β-(p-aminophényl)éthylamine et la solution est agitée pendant 15 h dans un tube scellé à température ambiante.

L'alkylglycoside est obtenu par l'addition de 0,2 ml d'alcool renfermant 3 mg de borohydrure de sodium, puis isolé par chromatographie sur Bio-gel P-2.

b) Couplage du dérivé oligosaccharide-phénéthylamine avec un protide.

A une solution de 10 µmol d'oligosaccharide-phénéthylamine dans 2 ml d'eau sont ajoutés successivement 0,8 ml d'HCL 0,1 N et 0,6 ml d'une solution de nitrite de sodium à 2 mg/ml.

Après 30 minutes de réaction, le mélange est lentement ajouté à 6 ml d'une solution de protéine (0,8 µmol) dans NaOH 0,05 N. Après 4 h de réaction, le produit de couplage est dialysé, puis purifié sur Bio-gel P-6.

Le rapport sucre : protéine peut atteindre 15 à 20.

II - Par couplage de glycopeptides et de protéines

100 mg de protéines sont dissous dans 10 ml de tampon borate de pH 9,5. A la solution refroidie à 0° C, on ajoute 0,2 ml de toluène-2,4 diisocyanate (TDIC). Le mélange est agité 30 minutes à 0° C, laissé une nuit à 4° C et centrifugé pour sédimenter l'excès de TDIC.

Le surnageant est recueilli, agité encore 1 h à 0° C, centrifugé si nécessaire, puis ajouté à 10 ml de tam pon borate pH 9,5 contenant 10 à 20 micromoles de glycopeptides. Le mélange est agité 1 h à 37° C, dialysé contre du carbonate d'ammonium 0,1 M (48 h), puis contre de l'eau distillée avant d'être lyophilisé.

La quantité de glycopeptides conjugués à la protéine est déterminée par un dosage colorimétrique des glucides.

Ce procédé est adapté de celui de Schick A.F. et Singer S.J. (J. Biol. Chem 236 (1961) 2477-2485).

**Revendications**

1.- Glycoprotéine extraite de l'hémocyanine d'un mollusque tel que Megathura crenulata (ou KLH), caractérisée en ce que sa fraction glucidique représente 5 à5,25 % de la molécule de glycoprotéine et en ce que la composition molaire de ladite fraction glucidique (calculée sur la base de 3 résidus de mannose) est sensiblement la suivant :

| | Résidus monosaccharidiques |
|---|---|
| Mannose | 3 |
| Galactose | 3 à 4 |
| Fucose | 2 à 3 |
| Glucose | 2 ± 0,2 |
| Xylose | 0,5 - 1 |
| N-acétylglucosamine | 3 - 4 |
| N-acétylgalactosamine | 2 ± 0,3 |

2.- Fraction glycopeptidique isolée de la glycoprotéine selon la Revendication 1, caractérisée en ce qu'elle est obtenue par protéolyse de la KLH suivie d'un traitement de purification du produit de protéolyse par chromatographie de gel-filtration, pour obtenir un produit purifié dont la composition en glucides (calculée sur la base de 3 résidus de mannose) est sensiblement la suivante :

| | Nbre/résidus monosaccharidiques |
|---|---|
| Mannose | 3 |
| Galactose | 4 |
| Fucose | 1,6 |
| Glucose | 4 |
| Xylose | 0,23 |
| N-acétylglucosamine | 3,3 |
| N-acétylgalactosamine | 2 |

3-. Fraction glycannique isolée de KLH selon la revendication 1 , caractérisée en ce qu'elle est obtenue par traitement alcalin de KLH ou des ses glycopeptides, suivie d'une N-réacétylation et de purifications sur Biogel P-2.

4.- Glycanne de synthèse, caractérisée en ce qu'il est sensiblement identique à la fraction glycannique selon la Revendication 3 et au glycanne de l'antigène 38 KD de S. mansoni et en ce qu'il présente au moins l'une des structures primaires suivantes :

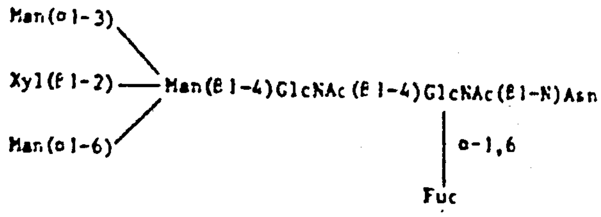

(I)

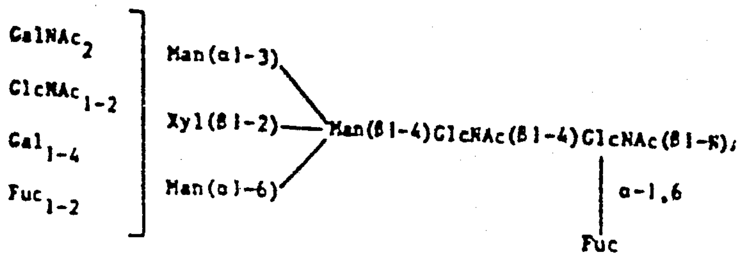

(II)

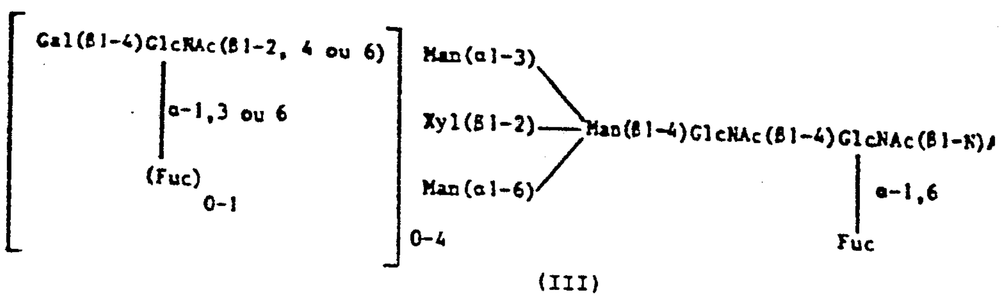

(III)

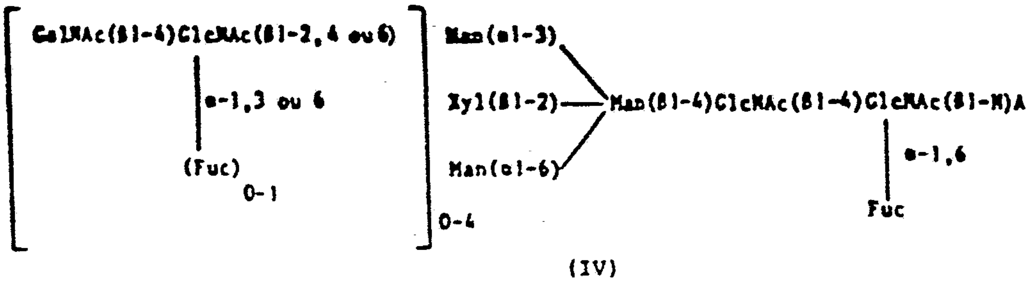

(IV)

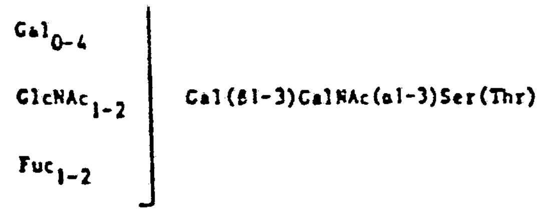

(V)

5-. Glycanne caractérisé en ce qu'il se compose de fucose, de galactose, de xylose, de mannose, de N-acétyl-glucosamine, de N-acétylgalactosamine et en ce qu'il présente la structure primaire (VI) suivante :

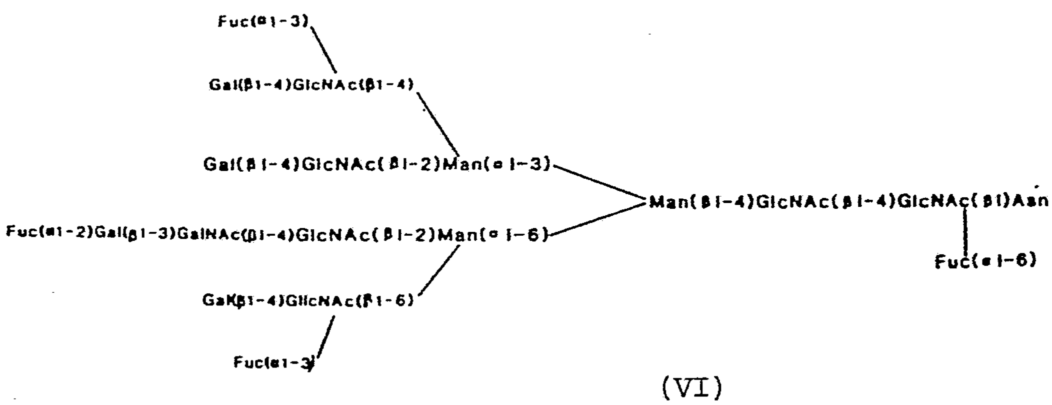

(VI)

6.- Glycanne selon l'une quelconque des Revendications 1, 3 et 4, caractérisé en ce qu'il est reconnu par un anticorps monoclonal spécifique de la molécule 38 KD.

7.- Sérum protecteur à l'égard des infections par Schistosoma mansoni, caractérisé en ce qu'il est constitué par un sérum de mammifère immunisé par la fraction glycoprotéique selon la Revendication 1 ou par son épitope glycannique selon l'un quelconque des Revendications 2 à 6.

8.- Composition immunogène, caractérisé en ce qu'elle contient en tant que constituant actif une quantité suffisante de la glycoprotéine selon la Revendication 1 ou de son épitope glycannique selon l'une quelconque des Revendications 2 à 6, éventuellement associé(e) à un adjuvant convenable.

9.- Vaccin contre les bilharzioses, caractérisé en ce qu'il contient une quantité vaccinante active de la glycoprotéine selon la Revendication 1 ou de son épitope glycannique selon l'une quelconque des Revendications 2 à 6.

10.- Agent immunisant, caractérisé en ce qu'il est constitué par un complexe formé par le couplage d'anticorps anti-idiotypes spécifiques d'anticorps monoclonaux antiSchistosoma mansoni, à la glycoprotéine selon la Revendication 1 ou à son epitope glycannique selon l'une quelconque des Revendications 2 à 4.

11.- Réactif de diagnostic apte à être utilisé pour la séroépidémiologie des bilharzioses, caractérisé en ce que son constituant principal est la glycoprotéine selon la Revendication 1 ou son épitope glycannique selon l'une quelconque des Revendications 2 à 4.

12.- Application de la glycoprotéine selon la Revendication 1 ou de son épitope glycannique selon l'une

quelconque des Revendications 2 à 4, à la préparation de néoglycoprotéines.

13.- Procédé de préparation de la glycoprotéine selon la Revendication 1, caractérisé en ce qu'il consiste à soumettre de l'hémocyanine de Megathura crenulata à au moins un traitement de protéolyse par la pronase et à isoler le produit de la protéolyse par précipitation par un agent précipitant approprié, tel que l'éthanol notamment.

14.- Procédé de préparation du glycopeptide selon la Revendication 2, caractérisé en ce que le produit de protéolyse isolé par précipitation conformément à la Revendication 13 est soumis à un traitement de purification approprié, de préférence à un traitement de purification par chromato graphie de gel-filtration, après quoi la fraction éluée présentant le pic majeur est isolée par précipitation, pour fournir une fraction glycopeptidique purifiée.

15.- Procédé de préparation d'un glycanne selon la Revendication 3, caractérisé en ce qu'il réalise l'hydrolyse alcaline de KLH ou de ses glycopeptides suivie d'une N-réacylation et d'une purification par chromatographie de tamisage moléculaire.

16. Procédé de préparation d'une néoglycoprotéine selon la Revendication 12, caractérisé en ce qu'un glycopeptide ou un glycanne selon l'une quelconque des Revendications 2, 3 ou 4, est condensé avec un peptide naturel ou synthétique approprié présentant une activité antigénique complémentaire de l'activité dudit glycanne.

17.- Procédé selon la Revendication 16, caractérisé en ce que la condensation desdits glycopeptides et glycannes avec des peptides appropriés est réalisée par couplage.

18.- Procédé selon l'une quelconque des Revendications 16 et 17, caractérisé en ce que le peptide est choisi dans le groupe qui comprend l'anatoxine tétanique et/ou des peptides de synthèse dérivés de la protéine 28kD .

19.- Néoglycoprotéines, caractérisées en ce qu'elles sont obtenues par application de la Revendication 12, en mettant en oeuvre le procédé selon l'une quelconque des Revendications 16 à 18.

# FIG. 1

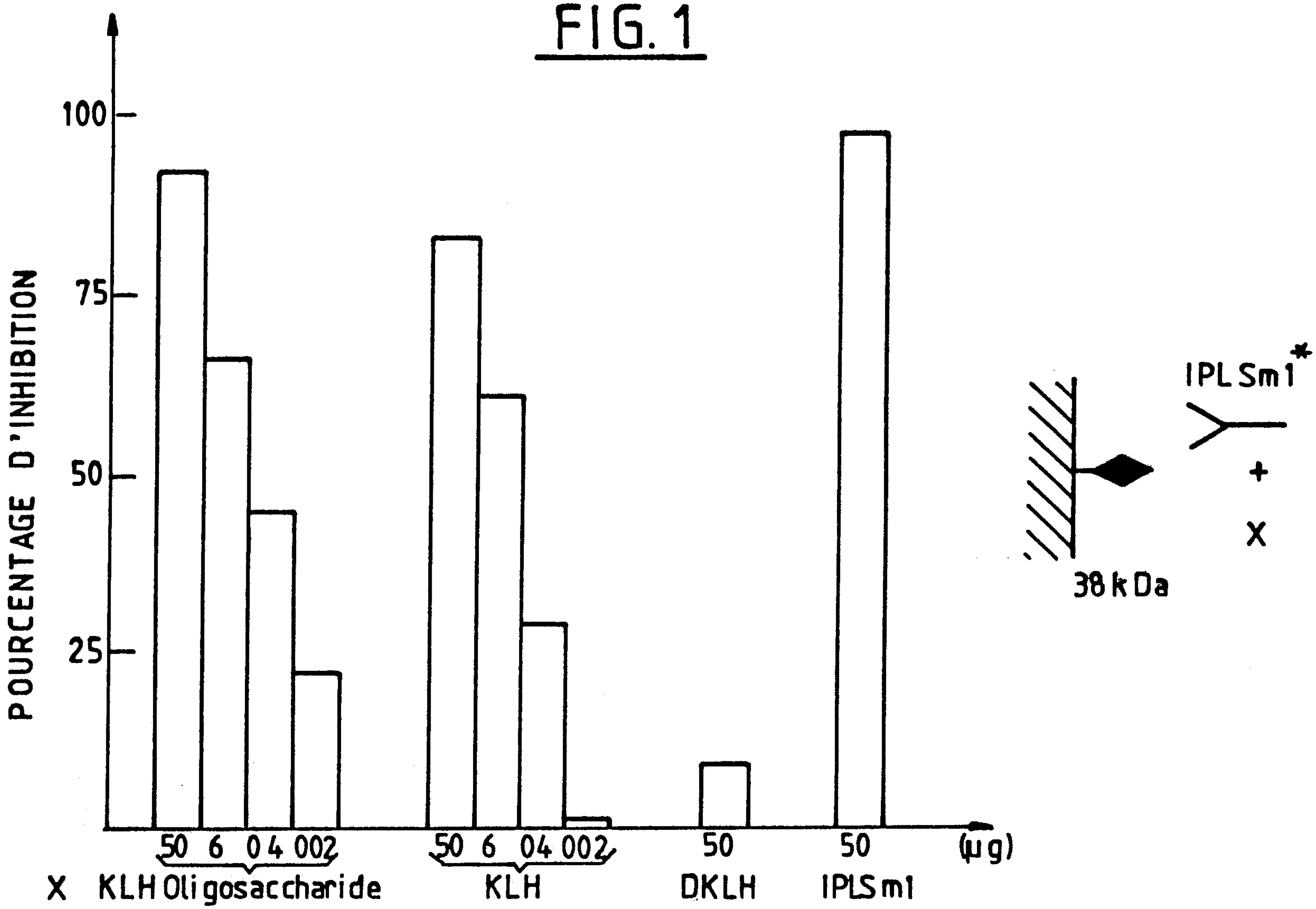

POURCENTAGE D'INHIBITION
100
75
50
25
50 6 04 002
X KLH Oligosaccharide
50 6 04 002
KLH
50
DKLH
50
IPLSm1
(µg)
IPLSm1*
+
X
38 kDa

FIG. 2

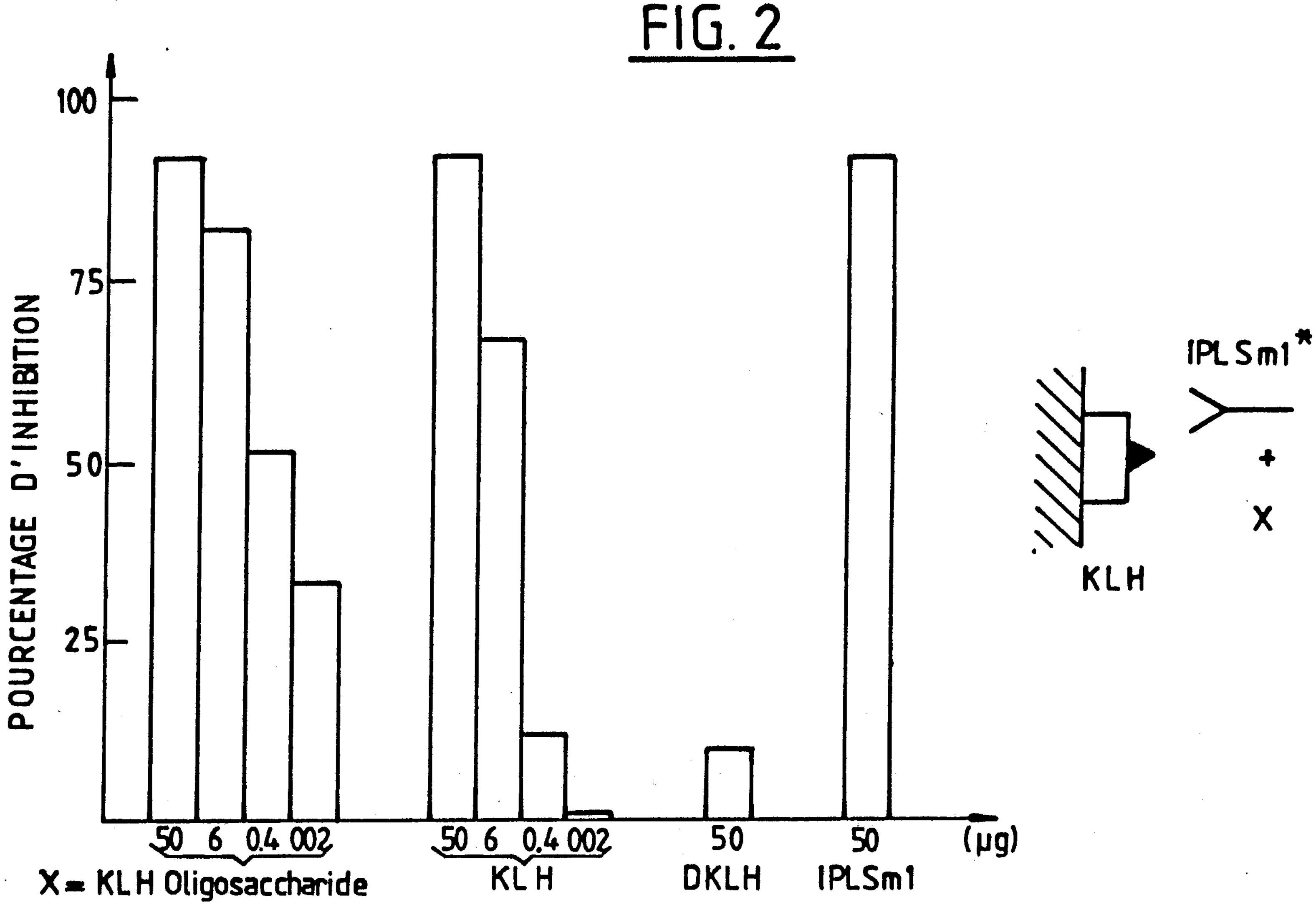
POURCENTAGE D'INHIBITION
100
75
50
25
50 6 0.4 002
X= KLH Oligosaccharide
50 6 0.4 002
KLH
50
DKLH
50
IPLSm1
(µg)
IPLSm1*
+
X
KLH

Office européen des brevets

# RAPPORT DE RECHERCHE EUROPEENNE

Numero de la demande

EP 87 40 1574

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X,P | BIOLOGICAL ABSTRACTS, vol. 83, no.12, 1987, résumé no. 118435, Philadelphia, US; J.-M. GRZYCH et al.: "Schistosoma mansoni shares a protective carbohydrate epitope with keyhole limpet hemocyanin", & EXP. MED. 165(3), 865-878, 1987<br>* Résumé *<br>--- | 1-19 | A 61 K 39/00<br>G 01 N 33/53 |
| X,P | NATURE, vol. 323, 2 octobre 1986, pages 443-445; C. DISSOUS et al.: "Schistosoma mansoni shares a protective oligosaccharide epitope with freshwater and marine snails"<br>* Article en entier *<br>--- | 1-19 | |
| A | DE-A-2 644 149 (BEHRINGWERKE AG)<br>----- | | **DOMAINES TECHNIQUES RECHERCHES (Int. Cl.4)**<br>A 61 K |

Le présent rapport de recherche a été établi pour toutes les revendications

| Lieu de la recherche | Date d'achèvement de la recherche | Examinateur |
|---|---|---|
| LA HAYE | 15-10-1987 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant